(19) **Europäisches Patentamt**
**European Patent Office**
**Office européen des brevets**

(11) **EP 4 252 650 B1**

(12) **EUROPEAN PATENT SPECIFICATION**

(45) Date of publication and mention
of the grant of the patent:
**10.12.2025 Bulletin 2025/50**

(51) International Patent Classification (IPC):
**A61B 5/319** (2021.01)    **A61B 5/367** (2021.01)
**A61B 5/00** (2006.01)    **G16H 50/00** (2018.01)

(21) Application number: **23156508.6**

(22) Date of filing: **14.02.2023**

(52) Cooperative Patent Classification (CPC):
**A61B 5/319; A61B 5/367; A61B 5/7267;
G16H 30/20; G16H 40/63; G16H 50/20;
G16H 50/30; G16H 50/70**

(54) **METHOD FOR GENERATING AN ACTIVATION MAP OF A PATIENT'S HEART**

VERFAHREN ZUR ERZEUGUNG EINER AKTIVIERUNGSKARTE DES HERZENS EINES
PATIENTEN

PROCÉDÉ DE GÉNÉRATION D'UNE CARTE D'ACTIVATION DU C UR D'UN PATIENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.03.2022 IT 202200004628**

(43) Date of publication of application:
**04.10.2023 Bulletin 2023/40**

(73) Proprietor: **XSPLINE S.P.A.**
**39100 Bolzano (IT)**

(72) Inventors:
• **KHAMZIN, Sviatoslav**
**39100 Bolzano (IT)**
• **CHMELEVSKY, Michail**
**39100 Bolzano (IT)**
• **RAINER, Werner**
**39100 Bolzano (IT)**

(74) Representative: **Studio Torta S.p.A.**
**Via Viotti, 9**
**10121 Torino (IT)**

(56) References cited:
**US-A1- 2021 193 291**

• **NEJIB ZEMZEMI ET AL: "From body surface
potential to activation maps on the atria: A
machine learning technique", COMPUTING IN
CARDIOLOGY (CINC), 2012, IEEE, 9 September
2012 (2012-09-09), pages 125 - 128,
XP032317071, ISBN: 978-1-4673-2076-4**
• **XU JINGJIA ET AL: "Noninvasive Transmural
Electrophysiological Imaging Based on
Minimization of Total-Variation Functional", IEEE
TRANSACTIONS ON MEDICAL IMAGING, IEEE,
USA, vol. 33, no. 9, 1 September 2014
(2014-09-01), pages 1860 - 1874, XP011557665,
ISSN: 0278-0062, [retrieved on 20140828], DOI:
10.1109/TMI.2014.2324900**
• **NEIC AUREL ET AL: "Efficient computation of
electrograms and ECGs in human whole heart
simulations using a reaction-eikonal model",
JOURNAL OF COMPUTATIONAL PHYSICS,
LONDON, GB, vol. 346, 15 June 2017
(2017-06-15), pages 191 - 211, XP085126085,
ISSN: 0021-9991, DOI: 10.1016/J.JCP.2017.06.020**

**Description**

CROSS-REFERENCE TO RELATED APPLICATIONS

This patent application claims priority from Italian patent application no. 102022000004628 filed on March 10, 2022.

TECHNICAL FIELD

The present invention relates to a method for generating an activation map of a patient's heart. Moreover, it relates to a respective computer program product, to a control unit configured to implement said method and to a device comprising said control unit. In particular, the method can be based on a generative adversarial network (GAN), it can implement a fully connected neural network as surrogate model for replacing the calculation of a forward model and it can include a reconstruction neural network to prevent mode collapse of the GAN.

BACKGROUND

As known, efficiently and easily recovering the cardiac ventricular activation sequence from ECG signals is crucial for performing several medical procedures and thus improving the patients' healthcare.
In particular, the ECG (electrocardiogram) signals acquired from a patient correspond to electric potentials measured on the patient's skin (e.g., on the skin of the torso of the patient); such electric potentials vary due to depolarization and repolarization of the heart and thus are indicative of the electrical activity of the cardiac muscle at each cardiac cycle (heartbeat). Recovering the cardiac ventricular activity from the ECG signals allows, for example, to perform more efficiently cardiac catheterization of the patient since the physician performing this procedure can be aware in advance of the specific conditions of the patient's heart and thus is able to place the catheter in the most appropriate regions of the heart (analogously, another application is the placing of pacemaker electrodes).
In the past, known solutions for obtaining an activation map (indicative of time propagation of an action potential wavefront in the myocardium) involved using invasive mapping techniques that are not based on acquisition of ECG signals but directly measure the electrical activity of the heart through electrodes placed in the heart or on its external surface. Nonetheless, this approach requires invasive access to the heart and has several serious limitations (e.g., high risk of local infection or thrombosis of the access site vessel) and risks for the patient's health that prevent it to be suitable for use in routine clinical practice.
Other known solutions involve non-invasive approaches based on acquisition of the ECG signals and calculation of activation map through mathematical models. In particular, these approaches exploit the solution of a so-called reverse problem, i.e. of a system of mathematical equations which starting from the ECG signals allows to obtain the activation map. Nonetheless, these approaches have several limitations. The main limitation is that the reverse problem is an ill-posed one that thus has several possible solutions, i.e. the accuracy of the method is not guaranteed. The number of possible solutions can be lowered by increasing the number of acquired electric signals; however, this requires using a large number of electrodes placed on the skin (e.g., hundreds of electrodes) and thus its use is impractical in routine clinical practice. Moreover, the accuracy of this approach is still very low and, for example, the error in identification of the heart regions that are electrically active continues to range from about 15mm to about 30mm. Furthermore, solving a reverse problem is extremely time consuming and has a high computational cost.
Document US 2021/193291 A1 relates to computer-based processes and procedures for reconstructing intracardiac electrical behaviour.

SUMMARY

The aim of the present invention is to provide a method for generating an activation map of a patient's heart, a respective computer program product, a control unit configured to implement said method and a device comprising said control unit that overcome the issues mentioned above.
According to the present invention, a method for generating an activation map of a patient's heart, a respective computer program product, a control unit configured to implement said method and a device comprising said control unit are provided, as defined in the annexed claims.

BRIEF DESCRIPTION OF THE DRAWINGS

For a better understanding of the present invention, preferred embodiments thereof are now described, purely by way of non-limiting example and with reference to the attached drawings, wherein:

- Figure 1 schematically shows a block-diagram of a device for implementing a method for generating an activation map of a heart of a patient, according to an embodiment;
- Figure 2 shows a 3D plot of the activation map of Figure 1, according to an embodiment;
- Figure 3 schematically shows a block-diagram of part of a control unit of the device of Figure 1, according to an embodiment; and
- Figure 4 is a block-diagram that schematically illustrates the method of Figure 1 for generating the activation map, according to an embodiment.

DESCRIPTION OF EMBODIMENTS

In particular, the figures are shown with reference to a triaxial Cartesian reference system defined by an X axis, a Y axis and a Z axis, orthogonal to each other.

In the following, elements common to the different embodiments have been indicated with the same reference numbers.

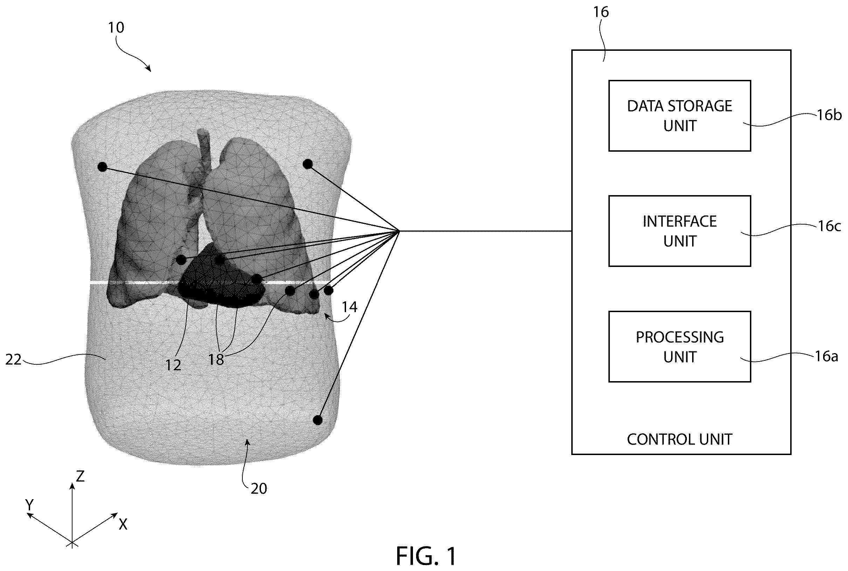

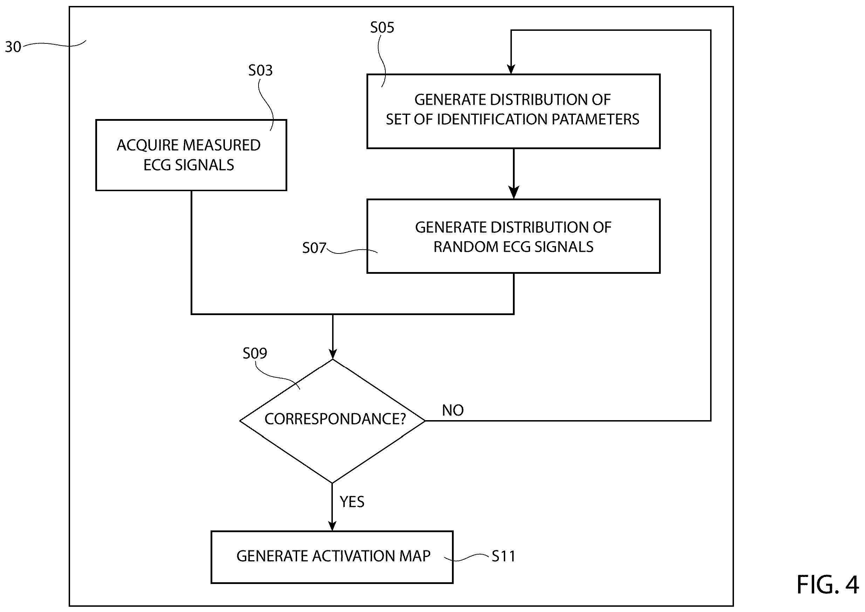

Figure 1 shows an activation map generation device 10 (in the following also called device 10) configured to implement an activation map generation method (also called for simplicity method and shown in Figure 4 with the reference number 30) for generating an activation map 40 of the heart 12 of a patient.

The activation map 40 is indicative of the time propagation of an action potential wavefront in the heart 12 (e.g., in the myocardium). In details, during its functioning, the heart 12 contracts and expands itself based on action potential signals (i.e., electric potentials, also called action potential wavefronts) induced by the nervous system of the patient; these contractions and expansions of the heart 12 are due to mechanical deformations (e.g., contractions and extensions) of cardiac muscle fibers of the myocardium, each of them being electrically controlled through these action potential signals. The activation of the cardiac muscle fibers is not simultaneous for all the cardiac muscle fibers: when an action potential signals is induced in a certain area of the heart 12, it spreads across the heart 12 by travelling across the cardiac muscle fibers (thus inducing their deformation). In other words, each action potential signal is transmitted through the heart 12 according to a time propagation that depends upon several factors (e.g., orientation of the cardiac muscle fibers, electrical conductivity of each region of the heart 12, pathologies of the heart 12, etc.). The time propagation of an action potential signal travelling through the heart 12 is described through said activation map 40.

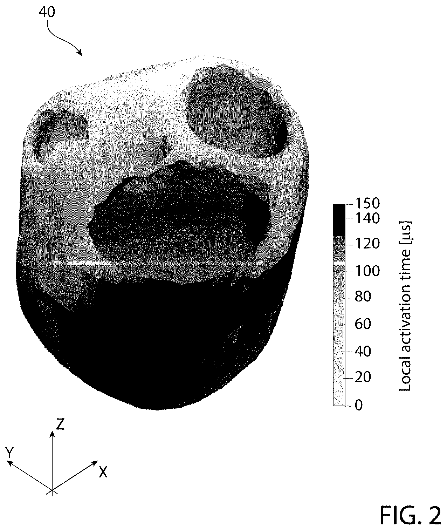

For illustration purposes, an example of an activation map 40 is shown in Figure 2. In particular, Figure 2 shows in greyscale the time progression of the wavefront of an action potential signal through the heart 12. In other words, it shows the local activation time of each region of the heart 12, i.e. the time delay necessary for said heart region to be activated by the action potential signal spreading from a starting region of the heart 12 (where the activation of the starting region of the heart 12 corresponds to t=0s). In details, in Figure 2 the local activation time increases as the colour darkens turning from white to black, i.e. the starting region of the heart 12 where the action potential signal is firstly applied is indicated in white while the regions of the heart 12 indicated in black are the last ones to receive the action potential signal wavefront after its travelling across the heart 12.

In particular, the activation map 40 is a map (e.g., a 3D map) in the form of a matrix (e.g., a 3D matrix) where each one of its cells is associated to a specific and respective portion of the heart 12 and has a value that is indicative of the local activation time of such respective portion of the heart 12.

Moreover, the action potential signals travelling through the heart 12 induce, due to known electrostatic effects, variations of an electrical potential measured on the skin 20 of the patient (in the following called skin potential). In particular, measured ECG signals indicative of the skin potential are acquired according to per se known ECG techniques and are dependent on the action potential signals according to per se known mathematical relationships (e.g., according to a so-called forward problem, better discussed in the following).

In the embodiment of Figure 1, the device 10 comprises a sensing module 14 and a control unit 16 operatively coupled between them. The sensing module 14 acquires physiological data (or physiological parameters) of the patient and the control unit 16 processes them to generate the activation map 40 according to the activation map generation method 30 described in the following.

In particular, the sensing module 14 comprises a plurality of ECG electrodes 18 that, during use, are attached in a removable way to the skin 20 of the patient to acquire the measured ECG signals. In details, the sensing module 14 comprises twelve ECG electrodes 18 placed on the skin 20 of the torso 22 of the patient and optionally on the skin of patient's limbs, in predefined positions that are well known in the electrocardiography field. Therefore, the physiological data of the patient at least comprise the measured ECG signals that are indicative of the skin potential variations caused by the action potential signals.

Optionally, the sensing module 14 further comprises other sensing elements to measure additional physiological data. For example, the sensing module 14 can further comprise medical imaging apparatuses for acquiring medical images of the patient or medical data obtained through medical imaging techniques, such as per se known patient's tetrahedral torso mesh (including labeled regions of the heart and lungs), myocardial fiber direction matrix, AHA ("American Heart Association") sectorization markers, universal ventricular coordinates (further details can be found for example in the prior art document https://www.sciencedirect.com/science/article/pii/S13618415 18300203), myocardial fiber direction field and myocardial tissue conductivity.

According to an exemplary and non-limiting embodiment, the control unit 16 comprises a processing unit 16a (such as a CPU, a microprocessor, an AP or a dedicated computing unit) for processing the physiological data and generating the activation map 40, and a data storage unit 16b (such as a memory, e.g. non-volatile) for storing data, coupled together. Optionally, the control unit 16 further comprises an interface unit 16c of a known type, coupled to the sensing module 14, to the processing unit 16a and to the data storage unit 16b and configured to pre-process the physiological data and/or electric signals acquired from the sensing module 14. For example, the interface unit 16c includes amplifier circuits and/or analog-to-digital converters (ADC) and/or serial communication modules (e.g., UART, SPI, I2C, etc.), not shown.

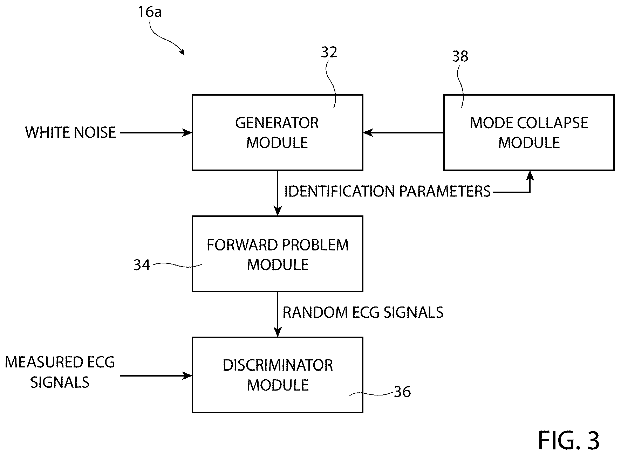

According to an embodiment described with reference to Figure 3, the control unit 16 (in details, the processing unit 16a) comprises a generator module 32, a forward problem module 34, a discriminator module 36 and, optionally, a collapse mode module 38, operatively coupled together and better described in the following.

In use, the device 10 implements the activation map generation method 30 for generating the activation map 40. The activation map generation method 30 is in particular performed by the control unit 16 and is described in the following with respect to Figure 4.

At a step S03 of the activation map generation method 30, the physiological data of the patient are acquired by the control unit 16 through the sensing module 14. In particular, in the following it is exemplarily considered the case in which the measured ECG signal is used in the activation map generation method 30; nevertheless, the activation map generation method 30 can analogously be executed by using more general ECG parameters indicative of the measured ECG signal (e.g., statistical parameters of the measured ECG signal such as variance, QRS duration of the ECG measured signal or parameters describing the ECG data, as disclosed for example in document Kuznetsov VV, Moskalenko VA, Gribanov DV and Zolotykh NY (2021) "Interpretable Feature Generation in ECG Using a Variational Autoencoder". Front. Genet. 12:638191. doi: 10.3389/fgene.2021.638191). Therefore, in the considered case at step S03 the control unit 16 acquires the measured ECG signal through the ECG electrodes 18 (i.e., acquires the ECG signal generated by combination of the electric signals measured by the ECG electrodes 18 acting as ECG derivations) . More in particular, the control unit 16 acquires a distribution of measured ECG signals, having an average correspondent to the measured ECG signal (actually measured by the ECG electrodes 18) and a variance related to the measurement noise.

At a step S05 of the activation map generation method 30, the generator module 32 of the control unit 16 generates, starting from white noise, a distribution of sets of identification parameters. Each set of identification parameters comprises a plurality of identification parameters and is correlated to a respective ECG signal (in the following named random ECG signal and better discussed in the following) and to a respective activation map (in the following named random activation map and better discussed in the following) through per se known mathematical relationships (in details, through a so-called forward problem better discussed in the following). In other words, a respective ECG signal and a respective activation map can be associated to each set of identification parameters through the forward problem.

According to an embodiment, the identification parameters comprise an activation source location $\Gamma$, an activation time $t_0$ and a conductivity parameter D. The activation source location $\Gamma$ is a vector in universal ventricular coordinates that is indicative of the location of the activation source in the heart 12 of a considered action potential signal (i.e., is indicative of the starting region of the heart 12); the activation time $t_0$ is a scalar coefficient in milliseconds that is indicative of the activation time at which the considered action potential signal is received by the activation source location in the heart 12; and the conductivity parameter D is a symmetric positive definite $3\times3$ tensor which is dependent on the myocardial fiber direction field and the myocardial tissue conductivity. As a non-limiting example, the activation source location $\Gamma$ ranges from (0.2, -2) to (0.8,-1) and is for example equal to about (0.3, -1.5) the activation time $t_0$ ranges from 0 ms to 20 ms and is for example equal to about 0.5 ms and the conductivity parameter D ranges from 0.05 to 1.5 and is for example equal to about 0.8 (unitless values, corresponding to respective physiological values of conduction velocity ranging from 1 cm/s to 150 cm/s and being for example equal to about 60 cm/s).

The distribution of sets of identification parameters is generated randomly starting from white noise, either according to per se known techniques or by means of machine learning techniques. In particular, according to an embodiment, the generator module 32 implements a machine learning algorithm trained to associate identification parameters to white noise. In details, the generator module 32 implements a generator (or generative network) of a generative adversarial network (GAN). GANs are well known supervised neural networks comprising a generator and a discriminator, as better discussed in the following. In particular, the generator module 32 implements a conditional GAN (cGAN; an example of which can be found in the document Mehdi Mirza, Simon Osindero, "Conditional Generative Adversarial Nets", 6 Nov 2014, arXiv:1411.1784 [cs.LG]); nevertheless, other GANs can analogously be used (examples can be found at link https://github.com/hindupuravinash/the-gan-zoo).

At a step S07 of the activation map generation method 30, consecutive to step S05, the forward problem module 34 of the control unit 16 acquires the distribution of sets of identification parameters and, based on them, generates a corresponding distribution of random ECG signals. In details, a respective random ECG signal is generated based on each set of identification parameters. Each random ECG signal is a signal, analogous to an ECG signal, which is not actually measured from the patient but is fictitiously generated as a function of randomly determined identification parameters.

Optionally, during generation of the random ECG signal, also a random activation map can be generated, depending on the used model or approach. The random activation map is an activation map, analogous to the activation map 40, which is not actually acquired from the patient through measurements or analysis but is fictitiously generated as a function of randomly determined identification parameters.

Optionally, the forward problem module 34 further receives as inputs additional data indicative of the patient's health and/or of patient's body structural features (e.g., the patient's torso mesh, the AHA sectorization markers, universal ventricular coordinates, health data of the patient such as age, sex, pathologies, etc.), for example acquired through the sensing module 14 or loaded in the control unit 16 either automatically or by medical staff. These additional data can be used by the forward problem module 34, in a per se known way, to improve the accuracy and patient specificity of the distribution of random ECG signals.

According to an embodiment, each random ECG signal is determined, based on the respective set of identification parameters, by solving a bidomain model of a per se known type (further details can be found for example in the document Muriel Boulakia, Serge Cazeau, Miguel Angel Fernández, Jean-Frédéric Gerbeau, Nejib Zemzemi. Mathematical Modeling of Electrocardiograms: A Numerical Study. Annals of Biomedical Engineering, Springer Verlag, 2010, 38 (3), pp.1071-1097. ff10.1007/s10439-009-9873-0ff. ffinria-00400490v2f). In details, the bidomain equations of the bidomain model allow to obtain both an ECG signal (i.e., the random ECG signal) and a corresponding activation map (i.e., the random activation map) for each set of identification parameters set as input.

According to a different embodiment, each random ECG signal is determined, based on the respective set of identification parameters, by solving both a monodomain model and a pseudo-bidomain model of per se known types (further details about the monodomain model can be found for example in the document Keener, J.P. An eikonal-curvature equation for action potential propagation in myocardium. J. Math. Biol. 29, 629-651 (1991). https://doi.org/10.1007/BF00163916, while further details about the pseudo-bidomain model can be found for example in the document Muriel Boulakia, Serge Cazeau, Miguel Angel Fernández, Jean-Frédéric Gerbeau, Nejib Zemzemi. Mathematical Modeling of Electrocardiograms: A Numerical Study. Annals of Biomedical Engineering, Springer Verlag, 2010, 38 (3), pp.1071-1097. ff10.1007/s10439-009-9873-0ff. ffinria-00400490v2f. In details, the monodomain model allows to obtain a respective activation map (i.e., the random activation map) from each set of identification parameters and, subsequently, the pseudo-bidomain model allows to obtain a respective ECG signal (i.e., the random ECG signal) from this random activation map.

According to a further embodiment, each random ECG signal is determined, based on the respective set of identification parameters, by solving a respective forward problem through solution of an Eikonal model of a per se known type (further details about the Eikonal model can be found for example in the document Pezzuto S, Kaľavský P, Potse M, Prinzen FW, Auricchio A and Krause R (2017) Evaluation of a Rapid Anisotropic Model for ECG Simulation. Front. Physiol. 8:265. doi: 10.3389/fphys.2017.00265 and of a pseudo-bidomain model (e.g., the same as previously discussed). In details, the Eikonal model allows to obtain a respective activation map (i.e., the random activation map) from each set of identification parameters and, subsequently, the pseudo-bidomain model allows to obtain a respective ECG signal (i.e., the random ECG signal) from this random activation map.

In details, the Eikonal model relies on an Eikonal equation to find the wavefront motion of an electrical wave during heart excitation (i.e., to find the propagation of the action potential signal and, thus, the activation map). In the Eikonal model, arrival times of an action potential wave front $t_a$ in a myocardial area $\Omega$ of the heart 12 define said activation map and are dependent both on a spatially inhomogeneous orthotropic velocity function indicative of the electrical conductivity in the myocardial area $\Omega$ (i.e., are dependent on the conductivity parameter $D=D(x)$) and on the activation source location $\Gamma$ at the activation time $t_0$. More in details, the Eikonal equation has the form:

$$\sqrt{\nabla t_a D \nabla t_a} = 1 \ in \ \Omega$$

$$t_a = t_0 \ in \ \Gamma$$

where $t_a$ is a positive function describing the wavefront arrival time at each location of the myocardial area $\Omega$ (i.e., is the random activation map).

Moreover, the pseudo-bidomain model relies on a lead-field approach of per se known type to calculate each random ECG signal according to the following mathematical relationship:

$$V(t) = \int_{\Omega} \nabla Z(x) \cdot G_i \nabla V_m(x, t) dx$$

where V(t) is the random ECG signal, $G_i$ is a predefined parameter indicative of the electrical conductivity of anatomical parts of the patient (other than the heart 12 and, for example, of the lungs, rib cage etc., where each anatomical part is indicated with a respective value of the index i, e.g. i=1,2,3..), $V_m(x,t)$ is a map of electric potentials in the myocardial area $\Omega$ in time and space (i.e., $V_m(x,t)$ is dependent on the arrival times of an action potential wave front $t_a$ in a myocardial area $\Omega$) and Z(x) is a lead field that is specific for each ECG electrode 18. More in details, the lead field Z(x) is the potential field created by a unit current applied to the ECG electrode 18 at location $x_j$ of the skin 20 and is obtained through the following mathematical relationship:

$$\nabla \cdot \left( G_i \nabla Z(x) \right) = \sum c_j \delta(x - x_j)$$

where $c_j$ are weight coefficients indicative of the reciprocal electrical contributions between the ECG electrodes 18 and $\delta$ is Dirac's delta function. This equation can be solved on the full torso mesh of the patient obtained from the sensing module 14. For example, to obtain cardiac potentials from the myocardial area $\Omega$, fixed potentials from a TNNP'06 cardiomyocyte model can be used.

According to a further embodiment, each random ECG signal is obtained, starting from the respective set of identification parameters, by means of a surrogate model based on machine learning techniques. In particular, the surrogate model is a mathematical method to replace complex mathematical calculations, by approximating a simpler model. More in details, the surrogate model is a machine learning algorithm that solves a regression problem to find random ECG signals corresponding to the input identification parameters. The surrogate model is a fully connected neural network trained on data that are generated when solving the forward problem by means of the bidomain model, or of the monodomain model and the pseudo-bidomain model, or of the Eikonal model and the pseudo-bidomain model. The loss function for the surrogate model can be the mean absolute error. Nonetheless, the fully connected neural network operating as surrogate model can be replaced with other machine learning techniques that solve the same regression problem (e.g., Gaussian process regression, Random Forest regression, polynomial regression etc.). Optionally, the surrogate model further receives as inputs the additional data indicative of the patient's health and/or of the patient's body structural features (e.g., the patient's torso mesh, the AHA sectorization markers, universal ventricular coordinates, health data of the patient such as age, sex, pathologies, etc.), as additional input features on which the surrogate model in trained and that the latter can use to further improve the regression accuracy.

At a step S09 of the activation map generation method 30, consecutive to step S07, the discriminator module 36 of the control unit 16 compares the distribution of measured ECG signals and the distribution of random ECG signals to verify if there is correspondence between them (e.g., if they coincide or if the discriminator does not distinguish between random ECG signals and measured ECG signals).

If there is correspondence, the activation map generation method 30 proceeds to a step S11, better discussed in the following.

On the other hand, if there is not correspondence, the activation map generation method 30 goes back to step S05 to repeat the steps S05-S09, thus verifying the correspondence between the measured ECG signals and further random ECG signals.

According to an embodiment, at the step S09 the discriminator module 36 compares the distribution of measured ECG signals and the distribution of random ECG signals according to per se known statistical techniques. For example, the discriminator module 36 calculates a cross-entropy between the distribution of measured ECG signals and of the distribution of random ECG signals and determines the correspondence between the distributions.

According to a different embodiment, at the step S09 the discriminator module 36 compares the distribution of measured ECG signals and the distribution of random ECG signals by means of machine learning techniques. In particular, the discriminator module 36 implements a machine learning algorithm trained to identify correspondence (i.e., a match) between the distribution of measured ECG signals and the distribution of random ECG signals. In details, the discriminator module 36 implements a discriminator (or discriminative network) of the GAN. For example, the discriminator receives as inputs the distribution of measured ECG signals and the distribution of random ECG signals and generates as output a probability value indicative of the probability that the two distributions correspond (e.g., coincide).

If there is correspondence between the distribution of measured ECG signals and the distribution of random ECG signals, the activation map 40 is generated by the control unit 16 at step S11.

According to an embodiment in which the forward problem module 34 generates at step S07 the distribution of random activation maps according to the distribution of sets of identification parameters provided by the generator module 32, the control unit 16 determines at step S11 the activation map 40 based on this distribution of random activation maps (i.e., based on the distribution of random activation maps related to the distribution of random ECG signals corresponding to the distribution of measured ECG signals). In details, the activation map 40 is calculated as a function of an average of these random activation maps (e.g., is equal to or proportional to said average), with a standard deviation that is the standard deviation of the distribution of random activation maps.

According to a different embodiment in which no distribution of random activation maps is generated at step S07 (e.g., in the case of the surrogate model), the control unit 16 generates at step S11 the activation map 40 based on the distribution of sets of identification parameters associated to the distribution of random ECG signals corresponding to the distribution of measured ECG signals. In details, the control unit 16 implements either the bidomain model, or the monodomain model and the pseudo-bidomain model, or the Eikonal model and the pseudo-bidomain model, using this distribution of sets of identification parameters to calculate a respective distribution of random activation maps (as previously described and thus not discussed again here); then, the activation map 40 is calculated as a function of an average of these random activation maps (e.g., is equal to or proportional to said average), with a standard deviation that is the standard deviation of the distribution of random activation maps.

Concerning the surrogate model and the GAN (of per se known type), formed by the generator implemented by the generator module 32 and by the discriminator implemented by the discriminator module 36, details about their trainings are now provided.

In particular, the surrogate model is firstly trained according to supervised techniques performed on a surrogate training dataset comprising a multiplicity of training sets of identification parameters (e.g., 10000 sets of parameters). For example, the training sets of identification parameters are generated by varying the activation source location $\Gamma$, the activation time $t_0$ and the conductivity parameter D in their ranges of variation through a Quasi-Monte Carlo Halton sampling of a per se known type, to create an evenly filled parametric space. Then, a respective multiplicity of forward problems are solved in order to determine a respective training random ECG signal for each training set of identification parameters (e.g., through the bidomain model, or the monodomain model and the pseudo-bidomain model, or the Eikonal model and the pseudo-bidomain model). The surrogate model is then trained according to per se known techniques based on the training sets of identification parameters and the training random ECG signals, so as to associate to each set of identification parameters the respective training random ECG signal. For example, the surrogate model is trained through the mean absolute error loss function to achieve a regression accuracy higher than about 95%.

Following to the training of the surrogate model (if present), the GAN is trained according to supervised techniques (e.g., for each patient). In details, the generator and the discriminator are trained simultaneously based on a GAN training dataset comprising a multiplicity of training white noise signals and training sets of identification parameters (e.g. 10000 sets) and a distribution of measured ECG signals (e.g., also comprising mean and variance of measured ECG signals). For example, the training white noise signals are generated randomly and are associated to respective training sets of identification parameters, while the distribution of measured ECG signals are acquired from measurements on the patient. In Nash equilibrium between the generator and the discriminator, the generator finds a patient-specific set of identification parameters of an electrophysiological model that allows to generate a respective activation map of this specific patient. In other words, the output of the discriminator (i.e., the correspondence or not between the random ECG signals and the measured ECG signals) is used as a feedback for the quality estimation of the training the GAN. More in details, white noise is associated with a respective set of identification parameters, which is associated with a respective random ECG signal. Thus, if the discriminator decides that the random ECG signals correspond with the measured ECG signals with some probability, it passes this probability to the generator. The generator updates its weights on the network to generate more realistic parameters, which can lead to more realistic random ECG signals. The discriminator, in turn, learns at each iteration to determine the probability that the incoming distribution matches the measured one, using the cross-entropy loss function.

For example, the generator is trained through the following loss function:

$$L_G = E_{Z \sim P_Z} log[D(G(z))] - E_{Z \sim P_Z} log[1 - D(G(z))] + K_{mc}$$

where G is the generator, z is the input white noise, $L_G$ is the loss of the generator, $K_{mc}$ is a collapse mode coefficient better discussed in the following and generally equal to zero, $E_Z$ is the expected value over all the random inputs provided to the generator, Pz is the distribution of the white noise, G(z) is the generator output when the white noise z is provided in input, D(G(z)) is the discriminator's estimate probability that the generated ECG signal corresponds to the measured ECG signal. Moreover, the discriminator is trained through the following loss function:

$$L_D = E_{Z \sim P_d} log[D(x)] + E_{Z \sim P_Z} log[1 - D(G(z))]$$

where D is the discriminator, $L_D$ is the loss of the discriminator, x is a sample of the measured ECG signals, $P_d$ is the distribution of measured ECG signals and D(x) is the discriminator's estimate probability that the generated ECG signal corresponds to the measured ECG signal..

For example, an Adam optimizer is used with step size of 0.0001 for G and with step size of 0.00004 for D; the $\beta_1$ and $\beta_2$ parameters of the Adam optimizer can be set to default values of about 0.9 and about 0.999 respectively.

Furthermore, training parameters of these neural network loss functions can be different, as described for example in the following prior art document: Sebastian Nowozin, Botond Cseke, Ryota Tomioka, "f-GAN: Training Generative Neural Samplers using Variational Divergence Minimization", 30th Conference on Neural Information Processing Systems (NIPS 2016), Barcelona, Spain.

Moreover, further details about methods and algorithms for calculating or pre-processing input data (e.g. muscle fibers, segmented heart, data about lungs, torso, other organs, forward models, cellular models) are described in these prior art documents: Lopez-Perez, A., Sebastian, R. & Ferrero, J.M. Three-dimensional cardiac computational modelling: methods, features and applications. BioMed Eng OnLine 14, 35 (2015). https://doi.org/10.1186/s12938-015-0033-5; Muriel Boulakia, Serge Cazeau, Miguel Angel Fernández, Jean-Frédéric Gerbeau, Nejib Zemzemi. Mathematical Modeling of Electrocardiograms: A Numerical Study. Annals of Biomedical Engineering, Springer Verlag, 2010, 38 (3), pp.1071-1097. ff10.1007/s10439-009-9873-0ff. ffinria-00400490v2; Pathmanathan P and Gray RA (2018) Validation and Trustworthiness of Multiscale Models of Cardiac Electrophysiology. Front. Physiol. 9:106. doi: 10.3389/fphys.2018.00106.

Moreover, optionally, the collapse mode module 38 can be present in the control unit 16 during training of the GAN. The collapse mode module 38 receives as inputs both the white noise input to the generator and the corresponding sets of identification parameters generated by the generator based on said white noise, and it implements machine learning techniques to prevent collapse mode of the generator during training. As known, collapse mode is a condition where GAN generators return only a small subset of all possible solutions (i.e., always return a same sample subgroup, smaller than the whole possible sample group). In details, the collapse mode module 38 implements a reconstruction neural network of a per se known type for avoiding the generator collapse mode. In particular, the reconstruction neural network acquires the sets of identification parameters generated by the generator, determines, based on the sets of identification parameters, if the generator of the GAN is in a condition indicative of mode collapse risk and, if the generator of the GAN is in said condition indicative of mode collapse risk, a setting of the generator of the GAN is modified to avoid mode collapse. In further details, the reconstruction neural network is trained to reconstruct white noise starting from the sets of identification parameters generated by the generator and to compare the reconstructed white noise with the original white noise set as input to the generator to generate these sets of identification parameters: if they correspond (e.g., coincide) there is no collapse mode risk, while if they do not correspond (e.g., they do not coincide) collapse mode risk is determined. In case collapse mode risk is determined, an additional term (the collapse mode coefficient $K_{mc}$, i.e. said modifiable setting of the generator of the GAN) is used to prevent collapse mode of the generator. In details, the collapse mode coefficient $K_{mc}$ is null when there is no collapse mode risk (i.e., the more the reconstructed white noise and the original white noise correspond between each other) and it is increased as the collapse mode risk is determined (i.e., said condition indicative of mode collapse risk is determined) and increases (in details, it increases the more as more the reconstructed white noise and the original white noise differ between each other) . For example, the collapse mode coefficient $K_{mc}$ can vary between 0 and about 100. In fact, by having the collapse mode coefficient $K_{mc}$ dependent on the collapse mode risk, the occurrence of collapse mode in the generator is avoided due to the use of this loss function dependent on the collapse mode coefficient $K_{mc}$. For example, the reconstruction neural network uses a squared loss that is indicative of the collapse mode risk and is calculated according to the following mathematical relationship:

$$L_R = E_{Z \sim P_g} \left\| z - R\big(G(z)\big) \right\|^2$$

where R is the reconstruction neural network, $L_R$ is the squared loss of the reconstruction neural network and $P_g$ is the distribution of the generated identification parameters and R(G(z)) is a reconstructed noise from the generated identification parameters. Usually $K_{mc}$ is equal to $L_R$, but in some cases a multiplier can be used to reduce the probability of collapse.

Other optional improvements to avoid collapse mode can also be implemented and are for example described in prior art document Akash Srivastava, Lazar Valkov, Chris Russell, Michael U. Gutmann, Charles Sutton, "VEEGAN: Reducing Collapse mode in GANs using Implicit Variational Learning", Published as a conference paper at NIPS, 2017, arXiv:1705.07761 and in prior art document Luke Metz, Ben Poole, David Pfau, Jascha Sohl-Dickstein, "Unrolled Generative Adversarial Networks", arXiv:1611.02163.

From what has been described and illustrated previously, the advantages of the present invention are evident.

The activation map generation method 30 allows to obtain the activation map 40 of the heart 12 of a patient (i.e., the time propagation of action potentials in the heart 12) starting from measured ECG signals and without the need to solve reverse problems that are highly time and power consuming.

On the other hand, the activation map generation method 30 allows to compare the measured ECG signals with random ECG signals fictitiously generated in a random way (through the solution of forward problems, which are less time and power consuming than the reverse problems) and, when correspondence is obtained, retrieve the activation maps associated to these random ECG signals to determine the activation map 40.

In other words, no reverse problems must be solved to determine the activation map 40 and no great amount of ECG electrodes are necessary, contrary to the known solutions.

Moreover, the GAN implemented in the activation map generation method 30 is robust against mode collapse due to the presence of the reconstruction neural network in the mode collapse module 38.

Therefore, the activation map generation method 30 is easy to be implemented, shows high classification accuracy and requires low computational costs and time.

Finally, it is clear that modifications and variations may be made to what has been described and illustrated herein, without thereby departing from the scope of the present invention, as defined in the annexed claims. For example, the different embodiments described can be combined with each other to provide further solutions.

Moreover, the sensing module 14 can be absent from the device 10. In this case, the control unit 16 receives the physiological data of the patient from external apparatuses or from configuration executed by medical staff (e.g., from external servers or through manual configuration by a physician) and processes them to generate the activation map 40.

The activation map generation method 30 has been described with reference to a distribution of sets of identification parameters, a distribution of random ECG signals, a distribution of measured ECG signals and a distribution of random activation maps. Nevertheless, the activation map generation method 30 can analogously be simplified to use (at each iteration of steps S05-S09) only one set of identification parameters, one random ECG signal, one measured ECG signal and one random activation map. In this case, the activation map 40 coincides with the random activation map corresponding to the set of identification parameters leading to the random ECG signal in correspondence with the measured ECG signal.

Moreover, the activation map generation method 30 has been described with reference to random ECG signals and measured ECG signals (e.g., input to the discriminator module 36). Nonetheless, the random ECG signals and measured ECG signals can be analogously replaced as inputs to the discriminator module 36 with more general random ECG parameters and, respectively, measured ECG parameters that are indicative of the random ECG signals and, respectively, of the measured ECG signals. For example, the random ECG parameters and the measured ECG parameters can be statistical parameters of the random ECG signals and, respectively, of the measured ECG signals, such as variance and/or peak-to-peak and/or QRS duration and/or parameters describing ECG data, further detail can be found in Kuznetsov VV, Moskalenko VA, Gribanov DV and Zolotykh NY (2021) Interpretable Feature Generation in ECG Using a Variational Autoencoder. Front. Genet. 12:638191. doi: 10.3389/fgene.2021.638191). For example, the random ECG parameters are calculated based on the random ECG signals and the measured ECG parameters are calculated based on the measured ECG signals and then the random ECG parameters and the measured ECG parameters are compared between each other by the discriminator module 36; otherwise, the forward problem module can directly output the random ECG parameters and the measured ECG parameters can be directly acquired by the control unit 16 instead of the measured ECG signals, so that the discriminator module 36 can compare the random ECG parameters and the measured ECG parameters. More in general, the activation map generation method 30 receives as inputs measured ECG data (either the measured ECG parameters, or the measured ECG signal or the distribution of measured ECG signals) that are compared by the discriminator module 36 with random ECG data (either the random ECG parameters, or the random ECG signal or the distribution of random ECG signals), received from the forward problem module 34, to verify correspondence.

Moreover, according to a different embodiment, the identification parameters can comprise the conductivity parameter D, a set of activation source locations $\Gamma$ and a respective set of activation times $t_0$. Each activation source location $\Gamma$ and respective activation time $t_0$ are relative to a respective action potential wavefront provided to a respective starting region of the heart 12.

## Claims

1. Method (30) for generating an activation map (40) indicative of a time propagation of an action potential wavefront in a heart (12) of a patient, the method (30) being executed by a control unit (16) and comprising the steps of:

   a. acquiring (S03) measured electrocardiography, ECG, data of the patient through a sensing module (14) operatively coupled to the control unit (16);
   b. generating (S05), by a generator module (32) of the control unit (16) and based on white noise, at least one set of identification parameters, each set of identification parameters identifying respective random ECG data and a respective random activation map that is indicative of a respective time propagation of a random action potential wavefront in the heart (12) of the patient;
   c. generating (S07), by a forward problem module (34) of the control unit (16) and based on each set of identification parameters, said respective random ECG data;
   d. comparing (S09), by a discriminator module (36) of the control unit (16), the random ECG data and the measured ECG data to determine if there is correspondence between them; and

e. if there is correspondence between the random ECG data and the measured ECG data, generating (S11) the activation map (40) based on the at least one random activation map determined based on the at least one set of identification parameters used to obtain the random ECG data in correspondence with the measured ECG data.

2. Method (30) according to claim 1, wherein the step of acquiring (S03) the measured ECG data comprises one of the following: acquiring a measured ECG signal from the patient; acquiring a distribution of measured ECG signals from the patient; acquiring measured ECG parameters indicative of a measured ECG signal or of a distribution of measured ECG signals acquired from the patient, and

wherein the step of generating (S07) the random ECG data respectively comprises one of the following: generating a random ECG signal; generating a distribution of random ECG signals; generating random ECG parameters indicative of a random ECG signal or of a distribution of random ECG signals, and
wherein, if the step of generating (S05) the at least one set of identification parameters comprises generating a distribution of sets of identification parameters, the step of generating (S07) the random ECG data comprises generating said distribution of random ECG signals, each set of identification parameters identifying one respective random ECG signal of the distribution of random ECG signals and one respective random activation map associated to said random ECG signal of the distribution of random ECG signals.

3. Method (30) according to claim 1 or 2, wherein the step of generating (S05) the at least one set of identification parameters comprises using a generator of a generative adversarial network, GAN, to receive white noise and to generate the at least one set of identification parameters, and
wherein the step of comparing (S09) the random ECG data and the measured ECG data comprises using a discriminator of said GAN, to receive the random ECG data and the measured ECG data and to generate a probability value indicative of a probability that the random ECG data and the measured ECG data correspond.

4. Method (30) according to anyone of the preceding claims, wherein each set of identification parameters comprises at least one activation source location ($\Gamma$), at least one activation time ($t_0$) and a conductivity parameter (D), wherein each activation source location ($\Gamma$) is indicative of a respective location in the heart (12) of a respective activation source of said action potential wavefront, each activation time ($t_0$) is indicative of a respective activation time at which said action potential wavefront is received at said location of the respective activation source in the heart (12), and the conductivity parameter (D) is indicative of a myocardial fiber direction field and of a myocardial tissue conductivity.

5. Method (30) according to anyone of the preceding claims, wherein the step of generating (S07) the random ECG data comprises receiving, by a surrogate model implemented by the forward problem module (34), the at least one set of identification parameters and generating, by the surrogate model, the respective random ECG data for each set of identification parameters, the surrogate model being based on machine learning techniques and being trained to associate the respective random ECG data to each set of identification parameters.

6. Method (30) according to claim 5, wherein the surrogate model is a fully connected neural network.

7. Method (30) according to claim 5 or 6, further comprising the step of solving a bidomain model or a monodomain model or an Eikonal model, to obtain the respective random activation map for each set of identification parameters.

8. Method (30) according to anyone of claims 1-4, wherein the step of generating (S07) the random ECG data comprises solving, based on each set of identification parameters, a bidomain model, or a monodomain model and a pseudo-bidomain model, or an Eikonal model and a pseudo-bidomain model, to obtain both the random ECG data and the random activation map.

9. Method (30) according to claims 2 and 7 or according to claims 2 and 8, wherein the step of generating (S05) the at least one set of identification parameters comprises generating a distribution of sets of identification parameters and the step of generating (S07) the random ECG data comprises generating said distribution of random ECG signals, and wherein the step of generating (S11) the activation map (40) comprises calculating an average of the random activation maps, the activation map (40) being determined as a function of said average.

10. Method (30) according to anyone of the preceding claims when depending on claim 3, further comprising the step of implementing, by a mode collapse module (38) of the control unit (16), a reconstruction neural network for:

- receiving as inputs the at least one set of identification parameters;
- determining, based on the at least one set of identification parameters, if the generator of the GAN is in a condition indicative of mode collapse risk; and
- if the generator of the GAN is in said condition indicative of mode collapse risk, modifying a setting of the generator of the GAN to avoid mode collapse.

11. Method (30) according to anyone of the preceding claims, wherein the step of generating (S07) the random ECG data comprises acquiring as inputs, by the forward problem module (34), additional data indicative of the patient's health and/or of patient's body structural features and generating the random ECG data also based on said additional data.

12. Method (30) according to anyone of the preceding claims, wherein the step of comparing (S09) the random ECG data and the measured ECG data to determine if there is correspondence between them comprises determining if the random ECG data and the measured ECG data coincide.

13. Method (30) according to anyone of the preceding claims, further comprising, if there is no correspondence between the random ECG data and the measured ECG data, repeating the steps b. to e.

14. Computer program product storable in a control unit (16), the computer program being designed so that, when executed, the control unit (16) becomes configured to cause the device of claim 16 to perform a method according to anyone of claims 1-13.

15. Control unit (16) for executing a method (30) for generating an activation map (40) indicative of a time propagation of an action potential wavefront in a heart (12) of a patient, the control unit (16) being configured to:

- acquire (S03) measured electrocardiography, ECG, data of the patient;
- generate (S05), by a generator module (32) of the control unit (16) and based on white noise, at least one set of identification parameters, each set of identification parameters identifying respective random ECG data and a respective random activation map that is indicative of a respective time propagation of a random action potential wavefront in the heart (12) of the patient;
- generate (S07), by a forward problem module (34) of the control unit (16) and based on each set of identification parameters, said respective random ECG data;
- compare (S09), by a discriminator module (36) of the control unit (16), the random ECG data and the measured ECG data to determine if there is correspondence between them; and
- if there is correspondence between the random ECG data and the measured ECG data, generate (S11) the activation map (40) based on the at least one random activation map determined based on the at least one set of identification parameters used to obtain the random ECG data in correspondence with the measured ECG data.

16. Device (10) for executing a method (30) for generating an activation map (40) indicative of a time propagation of an action potential wavefront in a heart (12) of a patient, the device (10) comprising a control unit (16) according to claim 15 and a sensing module (14) operatively coupled to the control unit (16) and configured to acquire the measured ECG data.

17. Device (10) according to claim 16, wherein the sensing module (14) comprises ECG electrodes (18) fixable, in a removable way, to a skin (20) of the patient and configured to sense of electrical potential variations on the skin (20) of the patient, the measured ECG data being indicative of said electrical potential variations.

**Patentansprüche**

1. Verfahren (30) zum Erzeugen einer Aktivierungskarte (40), die eine zeitliche Ausbreitung einer Aktionspotentialwellenfront in einem Herzen (12) eines/r Patienten/-in anzeigt, wobei das Verfahren (30) von einer Steuereinheit (16) ausgeführt wird und die folgenden Schritte umfasst:

a. Erfassen (S03) von gemessenen Elektrokardiographie-, EKG-, Daten des/r Patienten/- in durch ein Sensormodul (14), das betriebswirksam mit der Steuereinheit (16) gekoppelt ist;
b. Erzeugen (S05), durch ein Generatormodul (32) der Steuereinheit (16) und basierend auf weißem Rauschen, mindestens eines Satzes von Identifikationsparametern, wobei jeder Satz von Identifikationsparametern jeweilige Zufalls-EKG-Daten und eine jeweilige Zufalls-Aktivierungskarte identifiziert, die eine jeweilige zeitliche Ausbreitung einer Zufalls-Aktionspotentialwellenfront im Herzen (12) des/r Patienten/-in anzeigt;

c. Erzeugen (S07) der jeweiligen Zufalls-EKG-Daten durch ein Vorwärtsproblemmodul (34) der Steuereinheit (16) und auf der Grundlage jedes Satzes von Identifikationsparametern;

d. Vergleichen (S09) der Zufall-EKG-Daten und der gemessenen EKG-Daten durch ein Diskriminatormodul (36) der Steuereinheit (16), um zu bestimmen, ob eine Übereinstimmung zwischen ihnen besteht; und

e. wenn eine Übereinstimmung zwischen den Zufall-EKG-Daten und den gemessenen EKG-Daten besteht, Erzeugen (S11) der Aktivierungskarte (40) auf Grundlage der mindestens einen Zufalls-Aktivierungskarte, die auf Grundlage des mindestens einen Satzes von Identifikationsparametern bestimmt wird, die verwendet werden, um die Zufall-EKG-Daten in Übereinstimmung mit den gemessenen EKG-Daten zu erhalten.

2. Verfahren (30) nach Anspruch 1, wobei der Schritt des Erfassens (S03) der gemessenen EKG-Daten einen der folgenden Schritte umfasst: Erfassen eines gemessenen EKG-Signals von dem/r Patienten/-in; Erfassen einer Verteilung von gemessenen EKG-Signalen von dem/r Patienten/-in; Erfassen von gemessenen EKG-Parametern, die ein gemessenes EKG-Signal oder eine Verteilung von gemessenen EKG-Signalen, die von dem/r Patienten/-in erfasst wurden, anzeigen, und

wobei der Schritt des Erzeugens (S07) der Zufalls-EKG-Daten jeweils einen der folgenden Schritte umfasst: Erzeugen eines Zufalls-EKG-Signals; Erzeugen einer Verteilung von Zufalls-EKG-Signalen; Erzeugen von Zufalls-EKG-Parametern, die ein Zufalls-EKG-Signal oder eine Verteilung von Zufalls-EKG-Signalen anzeigen, und

wobei, wenn der Schritt des Erzeugens (S05) des mindestens einen Satzes von Identifikationsparametern das Erzeugen einer Verteilung von Sätzen von Identifikationsparametern umfasst, der Schritt des Erzeugens (S07) der Zufalls-EKG-Daten das Erzeugen der Verteilung von Zufalls-EKG-Signalen umfasst, wobei jeder Satz von Identifikationsparametern ein jeweiliges Zufalls-EKG-Signal der Verteilung von Zufalls-EKG-Signalen und eine jeweilige Zufalls-Aktivierungskarte, die dem Zufalls-EKG-Signal der Verteilung von Zufalls-EKG-Signalen zugeordnet ist, identifiziert.

3. Verfahren (30) nach Anspruch 1 oder 2, wobei der Schritt des Erzeugens (S05) des mindestens einen Satzes von Identifikationsparametern die Verwendung eines Generators eines generativen adversen Netzwerks, GAN, umfasst, um weißes Rauschen zu empfangen und den mindestens einen Satz von Identifikationsparametern zu erzeugen, und wobei der Schritt des Vergleichens (S09) der Zufall-EKG-Daten und der gemessenen EKG-Daten die Verwendung eines Diskriminators des GAN umfasst, um die Zufall-EKG-Daten und die gemessenen EKG-Daten zu empfangen und einen Wahrscheinlichkeitswert zu erzeugen, der eine Wahrscheinlichkeit anzeigt, dass die Zufall-EKG-Daten und die gemessenen EKG-Daten übereinstimmen.

4. Verfahren (30) nach einem der vorhergehenden Ansprüche, wobei jeder Satz von Identifikationsparametern mindestens einen Aktivierungsquellenort ($\Gamma$), mindestens eine Aktivierungszeit ($t_0$) und einen Leitfähigkeitsparameter (D) umfasst, wobei jeder Aktivierungsquellenort ($\Gamma$) einen jeweiligen Ort im Herzen (12) einer jeweiligen Aktivierungsquelle der Aktionspotentialwellenfront anzeigt, jede Aktivierungszeit ($t_0$) eine jeweilige Aktivierungszeit anzeigt, zu der die Aktionspotentialwellenfront an dem Ort der jeweiligen Aktivierungsquelle im Herzen (12) empfangen wird, und der Leitfähigkeitsparameter (D) ein Myokardfaserrichtungsfeld und eine Myokardgewebeleitfähigkeit anzeigt.

5. Verfahren (30) nach einem der vorhergehenden Ansprüche, wobei der Schritt des Erzeugens (S07) der Zufall-EKG-Daten das Empfangen des mindestens einen Satzes von Identifikationsparametern durch ein Surrogatmodell, das durch das Vorwärtsproblemmodul (34) implementiert wird, und das Erzeugen der jeweiligen Zufall-EKG-Daten für jeden Satz von Identifikationsparametern durch das Surrogatmodell umfasst, wobei das Surrogatmodell auf maschinellen Lerntechniken basiert und trainiert wird, um die jeweiligen Zufall-EKG-Daten jedem Satz von Identifikationsparametern zuzuordnen.

6. Verfahren (30) nach Anspruch 5, wobei das Surrogatmodell ein vollständig verbundenes neuronales Netzwerk ist.

7. Verfahren (30) nach Anspruch 5 oder 6, ferner umfassend den Schritt des Lösens eines Bidomänenmodells oder eines Monodomänenmodells oder eines Eikonalmodells, um die jeweilige Zufalls-Aktivierungskarte für jeden Satz von Identifikationsparametern zu erhalten.

8. Verfahren (30) nach einem der Ansprüche 1-4, wobei der Schritt des Erzeugens (S07) der Zufall-EKG-Daten das Lösen eines Bidomänenmodells oder eines Monodomänenmodells und eines Pseudo-Bidomänenmodells oder eines Eikonalmodells und eines Pseudo-Bidomänenmodells auf Grundlage jedes Satzes von Identifikationsparametern umfasst, um sowohl die Zufall-EKG-Daten als auch die Zufalls-Aktivierungskarte zu erhalten.

9. Verfahren (30) nach den Ansprüchen 2 und 7 oder nach den Ansprüchen 2 und 8, wobei der Schritt des Erzeugens (S05) des mindestens einen Satzes von Identifikationsparametern das Erzeugen einer Verteilung von Sätzen von Identifikationsparametern umfasst und der Schritt des Erzeugens (S07) der Zufalls-EKG-Daten das Erzeugen der Verteilung von Zufalls-EKG-Signalen umfasst, und
wobei der Schritt des Erzeugens (S11) der Aktivierungskarte (40) das Berechnen eines Durchschnitts der Zufalls-Aktivierungskarten umfasst, wobei die Aktivierungskarte (40) als eine Funktion des Durchschnitts bestimmt wird.

10. Verfahren (30) nach einem der vorhergehenden Ansprüche, wenn abhängig von Anspruch 3, ferner umfassend den Schritt des Implementierens eines neuronalen Rekonstruktionsnetzwerks durch ein Moduskollapsmodul (38) der Steuereinheit (16) zum:

- Empfangen des mindestens einen Satzes von Identifikationsparametern als Eingaben;
- Bestimmen auf Grundlage des mindestens einen Satzes von Identifikationsparametern, ob sich der Generator des GAN in einem Zustand befindet, der ein Moduskollapsrisiko anzeigt; und
- wenn sich der Generator des GAN in dem Zustand befindet, der ein Moduskollapsrisiko anzeigt, Ändern einer Einstellung des Generators des GAN, um einen Moduskollaps zu vermeiden.

11. Verfahren (30) nach einem der vorhergehenden Ansprüche, wobei der Schritt des Erzeugens (S07) der Zufall-EKG-Daten das Erfassen zusätzlicher Daten, die die Gesundheit des/r Patienten/-in und/oder die Körperstrukturmerkmale des/r Patienten/-in anzeigen, als Eingaben durch das Vorwärtsproblemmodul (34) und das Erzeugen der Zufall-EKG-Daten auch auf der Grundlage der zusätzlichen Daten umfasst.

12. Verfahren (30) nach einem der vorhergehenden Ansprüche, wobei der Schritt des Vergleichens (S09) der Zufall-EKG-Daten und der gemessenen EKG-Daten, um zu bestimmen, ob eine Übereinstimmung zwischen ihnen besteht, das Bestimmen umfasst, ob die Zufall-EKG-Daten und die gemessenen EKG-Daten übereinstimmen.

13. Verfahren (30) nach einem der vorhergehenden Ansprüche, ferner umfassend, wenn keine Übereinstimmung zwischen den Zufall-EKG-Daten und den gemessenen EKG-Daten besteht, das Wiederholen der Schritte b. bis e.

14. Computerprogrammprodukt, das in einer Steuereinheit (16) speicherbar ist, wobei das Computerprogramm so ausgelegt ist, dass, wenn es ausgeführt wird, die Steuereinheit (16) konfiguriert wird, um die Vorrichtung nach Anspruch 16 zu veranlassen, ein Verfahren nach einem der Ansprüche 1-13 durchzuführen.

15. Steuereinheit (16) zum Ausführen eines Verfahrens (30) zum Erzeugen einer Aktivierungskarte (40), die eine zeitliche Ausbreitung einer Aktionspotentialwellenfront in einem Herzen (12) eines/r Patienten/-in anzeigt, wobei die Steuereinheit (16) zu Folgendem konfiguriert ist:

- Erfassen (S03) von gemessenen Elektrokardiographie-, EKG-, Daten des/r Patienten/- in;
- Erzeugen (S05), durch ein Generatormodul (32) der Steuereinheit (16) und basierend auf weißem Rauschen, mindestens eines Satzes von Identifikationsparametern, wobei jeder Satz von Identifikationsparametern jeweilige Zufalls-EKG-Daten und eine jeweilige Zufalls-Aktivierungskarte identifiziert, die eine jeweilige zeitliche Ausbreitung einer Zufalls-Aktionspotentialwellenfront im Herzen (12) des/r Patienten/-in anzeigt;
- Erzeugen (S07) der jeweiligen Zufalls-EKG-Daten durch ein Vorwärtsproblemmodul (34) der Steuereinheit (16) und auf der Grundlage jedes Satzes von Identifikationsparametern;
- Vergleichen (S09) der Zufall-EKG-Daten und der gemessenen EKG-Daten durch ein Diskriminatormodul (36) der Steuereinheit (16), um zu bestimmen, ob eine Übereinstimmung zwischen ihnen besteht; und

wenn eine Übereinstimmung zwischen den Zufall-EKG-Daten und den gemessenen EKG-Daten besteht, Erzeugen (S11) der Aktivierungskarte (40) auf Grundlage der mindestens einen Zufalls-Aktivierungskarte, die auf Grundlage des mindestens einen Satzes von Identifikationsparametern bestimmt wird, die verwendet werden, um die Zufall-EKG-Daten in Übereinstimmung mit den gemessenen EKG-Daten zu erhalten.

16. Vorrichtung (10) zum Ausführen eines Verfahrens (30) zum Erzeugen einer Aktivierungskarte (40), die eine zeitliche Ausbreitung einer Aktionspotentialwellenfront in einem Herzen (12) eines/r Patienten/-in anzeigt, wobei die Vorrichtung (10) eine Steuereinheit (16) nach Anspruch 15 und ein Sensormodul (14) umfasst, das betriebswirksam mit der Steuereinheit (16) gekoppelt und konfiguriert ist, um die gemessenen EKG-Daten zu erfassen.

17. Vorrichtung (10) nach Anspruch 16, wobei das Sensormodul (14) EKG-Elektroden (18) umfasst, die entfernbar an einer Haut (20) des/r Patienten/-in befestigt werden können und konfiguriert sind, um elektrische Potentialschwankungen auf der Haut (20) des/r Patienten/-in zu detektieren, wobei die gemessenen EKG-Daten die elektrischen Potentialschwankungen anzeigen.

**Revendications**

1. Procédé (30) pour générer une carte d'activation (40) indiquant une propagation temporelle d'un front d'onde de potentiel d'action dans le cœur (12) d'un patient, le procédé (30) étant exécuté par une unité de commande (16) et comprenant les étapes suivantes :

   a. acquérir (S03) des données d'électrocardiographie ECG mesurées du patient par l'intermédiaire d'un module de détection (14) couplé de manière opérationnelle à l'unité de commande (16) ;
   b. générer (S05), par un module générateur (32) de l'unité de commande (16) et sur la base d'un bruit blanc, au moins un ensemble de paramètres d'identification, chaque ensemble de paramètres d'identification identifiant des données ECG aléatoires respectives et une carte d'activation aléatoire respective indiquant une propagation temporelle respective d'un front d'onde de potentiel d'action aléatoire dans le cœur (12) du patient ;
   c. générer (S07), par un module de problème direct (34) de l'unité de commande (16) et sur la base de chaque ensemble de paramètres d'identification, lesdites données ECG aléatoires respectives ;
   d. comparer (S09), par un module de discrimination (36) de l'unité de commande (16), les données ECG aléatoires et les données ECG mesurées pour déterminer s'il existe une correspondance entre elles ; et
   e. s'il y a correspondance entre les données ECG aléatoires et les données ECG mesurées, générer (S11) la carte d'activation (40) sur la base de l'au moins une carte d'activation aléatoire déterminée sur la base d'au moins un ensemble de paramètres d'identification utilisés pour obtenir les données ECG aléatoires en correspondance avec les données ECG mesurées.

2. Procédé (30) selon la revendication 1, dans lequel l'étape d'acquisition (S03) des données ECG mesurées comprend l'un des éléments suivants : acquérir un signal ECG mesuré du patient ; acquérir une distribution de signaux ECG mesurés du patient ; acquérir des paramètres ECG mesurés indicatifs d'un signal ECG mesuré ou d'une distribution de signaux ECG mesurés acquis du patient, et

   dans lequel l'étape de génération (S07) des données ECG aléatoires comprend respectivement l'une des étapes suivantes : générer un signal ECG aléatoire ; générer une distribution de signaux ECG aléatoires ; générer des paramètres ECG aléatoires indicatifs d'un signal ECG aléatoire ou d'une distribution de signaux ECG aléatoires, et
   dans lequel, si l'étape de génération (S05) de l'au moins un ensemble de paramètres d'identification consiste à générer une distribution d'ensembles de paramètres d'identification, l'étape de génération (S07) des données ECG aléatoires consiste à générer ladite distribution de signaux ECG aléatoires, chaque ensemble de paramètres d'identification identifiant un signal ECG aléatoire respectif de la distribution de signaux ECG aléatoires et une carte d'activation aléatoire respective associée audit signal ECG aléatoire de la distribution de signaux ECG aléatoires.

3. Procédé (30) selon la revendication 1 ou 2, dans lequel l'étape de génération (S05) de l'au moins un ensemble de paramètres d'identification comprend l'utilisation d'un générateur d'un réseau adversarial génératif GAN pour recevoir un bruit blanc et générer l'au moins un ensemble de paramètres d'identification, et
   dans lequel l'étape de comparaison (S09) des données ECG aléatoires et des données ECG mesurées consiste à utiliser un discriminateur dudit GAN pour recevoir les données ECG aléatoires et les données ECG mesurées et pour générer une valeur de probabilité indiquant une probabilité de correspondance entre les données ECG aléatoires et les données ECG mesurées.

4. Procédé (30) selon l'une quelconque des revendications précédentes, dans lequel chaque ensemble de paramètres d'identification comprend au moins un emplacement de source d'activation ($\Gamma$), au moins un temps d'activation ($t_0$) et un paramètre de conductivité (D),
   dans lequel chaque emplacement de source d'activation ($\Gamma$) indique un emplacement respectif dans le cœur (12) d'une source d'activation respective dudit front d'onde de potentiel d'action, chaque temps d'activation ($t_0$) indique un

temps d'activation respectif auquel ledit front d'onde de potentiel d'action est reçu audit emplacement de la source d'activation respective dans le cœur (12), et le paramètre de conductivité (D) indique un champ de direction de fibre myocardique et une conductivité de tissu myocardique.

5. Procédé (30) selon l'une quelconque des revendications précédentes, dans lequel l'étape de génération (S07) des données ECG aléatoires consiste à recevoir, par un modèle de substitution mis en œuvre par le module de problème direct (34), l'au moins un ensemble de paramètres d'identification et à générer, par le modèle de substitution, les données ECG aléatoires respectives pour chaque ensemble de paramètres d'identification, le modèle de substitution étant basé sur des techniques d'apprentissage automatique et étant entraîné à associer les données ECG aléatoires respectives à chaque ensemble de paramètres d'identification.

6. Procédé (30) selon la revendication 5, dans lequel le modèle de substitution est un réseau neuronal entièrement connecté.

7. Procédé (30) selon la revendication 5 ou 6, comprenant en outre l'étape de résolution d'un modèle bidomaine ou d'un modèle monodomaine ou d'un modèle éikonal, afin d'obtenir la carte d'activation aléatoire respective pour chaque ensemble de paramètres d'identification.

8. Procédé (30) selon l'une quelconque des revendications 1 à 4, dans lequel l'étape de génération (S07) des données ECG aléatoires consiste à résoudre, sur la base de chaque ensemble de paramètres d'identification, un modèle bidomaine, ou un modèle monodomaine et un modèle pseudo-bidomaine, ou un modèle éikonal et un modèle pseudo-bidomaine, pour obtenir à la fois les données ECG aléatoires et la carte d'activation aléatoire.

9. Procédé (30) selon les revendications 2 et 7 ou selon les revendications 2 et 8, dans lequel l'étape de génération (S05) de l'au moins un ensemble de paramètres d'identification comprend la génération d'une distribution d'ensembles de paramètres d'identification et l'étape de génération (S07) des données ECG aléatoires comprend la génération de ladite distribution de signaux ECG aléatoires, et
l'étape consistant à générer (S11) la carte d'activation (40) consiste à calculer une moyenne des cartes d'activation aléatoires, la carte d'activation (40) étant déterminée en fonction de ladite moyenne.

10. Procédé (30) selon l'une quelconque des revendications précédentes lorsqu'il dépend de la revendication 3, comprenant en outre l'étape consistant à mettre en œuvre, par un module d'effondrement de mode (38) de l'unité de commande (16), un réseau neuronal de reconstruction pour :

- recevoir en tant qu'entrées l'au moins un ensemble de paramètres d'identification ;
- déterminer, sur la base de l'au moins un ensemble de paramètres d'identification, si le générateur du GAN est dans un état indiquant un risque d'effondrement de mode ; et
- si le générateur du GAN est dans ledit état indiquant un risque d'effondrement de mode, modifier un réglage du générateur du GAN pour éviter l'effondrement de mode.

11. Procédé (30) selon l'une quelconque des revendications précédentes, dans lequel l'étape consistant à générer (S07) les données ECG aléatoires comprend l'acquisition en tant qu'entrées, par le module de problème direct (34), de données supplémentaires indiquant l'état de santé du patient et/ou les caractéristiques structurelles du corps du patient, et à générer les données ECG aléatoires également sur la base desdites données supplémentaires.

12. Procédé (30) selon l'une quelconque des revendications précédentes, dans lequel l'étape consistant à comparer (S09) les données ECG aléatoires et les données ECG mesurées pour déterminer s'il existe une correspondance entre elles consiste à déterminer si les données ECG aléatoires et les données ECG mesurées coïncident.

13. Procédé (30) selon l'une quelconque des revendications précédentes, comprenant en outre, s'il n'y a pas de correspondance entre les données ECG aléatoires et les données ECG mesurées, la répétition des étapes b. à e.

14. Produit de programme informatique stockable dans une unité de commande (16), le programme informatique étant conçu de telle sorte que, lorsqu'il est exécuté, l'unité de commande (16) devient configurée pour amener le dispositif de la revendication 16 à exécuter un procédé selon l'une quelconque des revendications 1 à 13.

15. Unité de commande (16) pour l'exécution d'un procédé (30) de génération d'une carte d'activation (40) indiquant la propagation temporelle d'un front d'onde de potentiel d'action dans le cœur (12) d'un patient, l'unité de commande (16) étant configurée pour :

- acquérir (S03) les données d'électrocardiographie ECG du patient ;
- générer (S05), par un module générateur (32) de l'unité de commande (16) et sur la base d'un bruit blanc, au moins un ensemble de paramètres d'identification, chaque ensemble de paramètres d'identification identifiant des données ECG aléatoires respectives et une carte d'activation aléatoire respective qui est indicative d'une propagation temporelle respective d'un front d'onde de potentiel d'action aléatoire dans le cœur (12) du patient ;
- générer (S07), par l'intermédiaire d'un module de problème direct (34) de l'unité de commande (16) et sur la base de chaque ensemble de paramètres d'identification, lesdites données ECG aléatoires respectives ;
- comparer (S09), par un module de discrimination (36) de l'unité de commande (16), les données ECG aléatoires et les données ECG mesurées pour déterminer s'il existe une correspondance entre elles ; et

s'il y a une correspondance entre les données ECG aléatoires et les données ECG mesurées, générer (S11) la carte d'activation (40) sur la base de l'au moins une carte d'activation aléatoire déterminée sur la base de l'au moins un ensemble de paramètres d'identification utilisés pour obtenir les données ECG aléatoires en correspondance avec les données ECG mesurées.

16. Dispositif (10) pour l'exécution d'un procédé (30) de génération d'une carte d'activation (40) indiquant la propagation temporelle d'un front d'onde de potentiel d'action dans le cœur (12) d'un patient, le dispositif (10) comprenant une unité de commande (16) selon la revendication 15 et un module de détection (14) couplé de manière opérationnelle à l'unité de commande (16) et configuré pour acquérir les données mesurées de l'ECG.

17. Dispositif (10) selon la revendication 16, dans lequel le module de détection (14) comprend des électrodes ECG (18) pouvant être fixées, de manière amovible, sur la peau (20) du patient et configurées pour détecter les variations de potentiel électrique sur la peau (20) du patient, les données ECG mesurées étant indicatives desdites variations de potentiel électrique.

FIG. 1

Local activation time [µs]

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102022000004628
- US 2021193291 A1

**Non-patent literature cited in the description**

- **KUZNETSOV VV** ; **MOSKALENKO VA** ; **GRIBANOV DV** ; **ZOLOTYKH NY**. Interpretable Feature Generation in ECG Using a Variational Autoencoder. *Front. Genet.*, 2021, vol. 12, 638191
- **MEHDI MIRZA** ; **SIMON OSINDERO**. Conditional Generative Adversarial Nets. *arXiv:1411.1784*, 06 November 2014
- Mathematical Modeling of Electrocardiograms: A Numerical Study. **MURIEL BOULAKIA** ; **SERGE CAZEAU** ; **MIGUEL ANGEL FERNÁNDEZ** ; **JEAN-FRÉDÉRIC GERBEAU** ; **NEJIB ZEMZEMI**. Annals of Biomedical Engineering. Springer Verlag, 2010, vol. 38, 1071-1097
- **KEENER, J.P.** An eikonal-curvature equation for action potential propagation in myocardium.. *J. Math. Biol.*, 1991, vol. 29, 629-651, https://doi.org/10.1007/BF00163916
- Mathematical Modeling of Electrocardiograms: A Numerical Study.. **MURIEL BOULAKIA** ; **SERGE CAZEAU** ; **MIGUEL ANGEL FERNÁNDEZ** ; **JEAN-FRÉDÉRIC GERBEAU** ; **NEJIB ZEMZEMI**. Annals of Biomedical Engineering. Springer Verlag, 2010, vol. 38, 1071-1097
- **PEZZUTO S** ; **KAL'AVSKÝ P** ; **POTSE M** ; **PRINZEN FW** ; **AURICCHIO A** ; **KRAUSE R**. Evaluation of a Rapid Anisotropic Model for ECG Simulation. *Front. Physiol.*, 2017, vol. 8, 265
- **SEBASTIAN NOWOZIN** ; **BOTOND CSEKE** ; **RYOTA TOMIOKA**. f-GAN: Training Generative Neural Samplers using Variational Divergence Minimization. *30th Conference on Neural Information Processing Systems*, 2016
- **LOPEZ-PEREZ, A.** ; **SEBASTIAN, R.** ; **FERRERO, J.M.** Three-dimensional cardiac computational modelling: methods, features and applications.. *BioMed Eng OnLine*, 2015, vol. 14, 35, https://doi.org/10.1186/s12938-015-0033-5
- **PATHMANATHAN P** ; **GRAY RA**. Validation and Trustworthiness of Multiscale Models of Cardiac Electrophysiology. *Front. Physiol.*, 2018, vol. 9, 106
- **AKASH SRIVASTAVA** ; **LAZAR VALKOV** ; **CHRIS RUSSELL** ; **MICHAEL U. GUTMANN** ; **CHARLES SUTTON**. VEEGAN: Reducing Collapse mode in GANs using Implicit Variational Learning. *arXiv:1705.07761*, 2017
- **LUKE METZ** ; **BEN POOLE** ; **DAVID PFAU** ; **JASCHA SOHL-DICKSTEIN**. Unrolled Generative Adversarial Networks. *arXiv:1611.02163*